# EUROPÄISCHE PATENTANMELDUNG

(11) **EP 4 059 916 A1**
(43) Veröffentlichungstag der Anmeldung: **21.09.2022**
(21) Anmeldenummer: 21162664.3
(22) Anmeldetag: 15.03.2021
(51) Int. Cl.: C07C 5/48, C07C 11/04, C07C 51/215, C07C 53/08

(54) **VERFAHREN UND ANLAGE ZUR ERZEUGUNG EINES PRODUKTKOHLENWASSERSTOFFS**

(71) Anmelder: Linde GmbH, 82049 Pullach (DE)
(72) Erfinder: Zellhuber, Mathieu, 82152 Martinsried (DE); Schubert, Martin, 81375 München (DE); Meiswinkel, Andreas, 83253 Rimsting (DE); Wöhl, Anina, 81379 München (DE)
(74) Vertreter: DehnsGermany Partnerschaft von Patentanwälten

(57) **Zusammenfassung**

Es wird ein Verfahren zur Erzeugung eines Produktkohlenwasserstoffs vorgeschlagen, bei dem ein Einsatzkohlenwasserstoff in einem den Einsatzkohlenwasserstoff und Sauerstoff enthaltenden Einsatzgemisch unter Erhalt eines den Produktkohlenwasserstoff und Wasser enthaltenden Produktgemischs einer Selektivoxidation unterworfen wird, und bei dem unter Abtrennung zumindest eines Teils des Wassers aus zumindest einem Teil des Produktgemischs ein Folgegemisch gebildet wird. Hierbei ist vorgesehen, dass der in dem Einsatzgemisch enthaltene Sauerstoff bei der Selektivoxidation teilweise umgesetzt wird, so dass das Produktgemisch einen ersten Restsauerstoffgehalt und das Folgegemisch einen zweiten Restsauerstoffgehalt aufweist. Eine Erfassung des ersten und/oder des zweiten Restsauerstoffgehalts wird unter Verwendung zumindest einer ersten Messeinrichtung vorgenommen. Am Ende des Katalysatorbetts ist zumindest eine zweite Messeinrichtung bereitgestellt, die zur Erfassung einer eine Betriebsstabilität kennzeichnenden Größe eingerichtet ist. Unter Verwendung einer Prozesssteuerungs- und/oder Auswerteeinheit werden über einen Erfassungszeitraum Messdaten der wenigstens einen ersten Messeinrichtung und/oder der wenigstens einen zweiten Messeinrichtung erfasst und unter Erhalt von Folgedaten ausgewert und/oder verarbeitet, und auf Grundlage der Folgedaten wird eine Verfahrenssteuerung durchgeführt. Eine entsprechende Anlage ist ebenfalls Gegenstand der vorliegenden Erfindung.

## Beschreibung

Die vorliegende Erfindung betrifft ein Verfahren und eine Anlage zur Herstellung eines Produktkohlenwasserstoffs gemäß den Oberbegriffen der entsprechenden unabhängigen Patentansprüche.

### Hintergrund der Erfindung

Die oxidative Dehydrierung (engl. Oxidative Dehydrogenation, ODH) von Paraffinen mit zwei bis vier Kohlenstoffatomen ist grundsätzlich bekannt. Bei der ODH werden die genannten Paraffine mit Sauerstoff unter anderem zu den jeweiligen Olefinen und zu Wasser umgesetzt. Die vorliegende Erfindung betrifft insbesondere die oxidative Dehydrierung von Ethan zu Ethylen, nachfolgend auch als ODHE bezeichnet, kann aber auch zur Herstellung anderer Olefine durch oxidative Dehydrierung entsprechender Paraffine und in anderen Verfahren zur selektiven katalytischen Oxidation von Kohlenwasserstoffen eingesetzt werden. Lediglich der Einfachheit halber wird nachfolgend hauptsächlich auf die ODHE Bezug genommen.

Die ODH kann gegenüber etablierteren Verfahren zur Herstellung von Olefinen wie dem Steamcracken oder der katalytischen Dehydrierung vorteilhaft sein. So besteht aufgrund der Exothermie der beteiligten Reaktionen und der praktisch irreversiblen Wasserbildung keine thermodynamische Gleichgewichtslimitierung. Die ODH kann bei vergleichsweise geringen Reaktionstemperaturen durchgeführt werden. Grundsätzlich ist keine Regenerierung der eingesetzten Katalysatoren erforderlich, da die Anwesenheit von Sauerstoff eine in-situ-Regenerierung ermöglicht bzw. bewirkt. Schließlich werden im Gegensatz zum Steamcracken geringere Mengen an wertlosen Nebenprodukten wie Koks gebildet.

Zu weiteren Details bezüglich der ODH sei auf einschlägige Fachliteratur, beispielsweise Ivars, F. und Lopez Nieto, J. M., Light Alkanes Oxidation: Targets Reached and Current Challenges, in: Duprez, D. und Cavani, F. (Hrsg.), Handbook of Advanced Methods and Processes in Oxidation Catalysis: From Laboratory to Industry, London 2014: Imperial College Press, Seiten 767-834, oder Gärtner, C.A. et al., Oxidative Dehydrogenation of Ethane: Common Principles and Mechanistic Aspects, ChemCatChem, Bd. 5, Nr. 11, 2013, Seiten 3196 bis 3217, sowie X. Li, E. Iglesia, Kinetics and Mechanism of Ethane Oxidation to Acetic Acid on Catalysts Based on Mo-V-Nb Oxides, J. Phys. Chem. C, 2008, 112, 15001-15008, verwiesen.

Bei der ODH werden insbesondere MoVNbOₓ- und MoVNbTeOₓ-basierte Katalysatoren eingesetzt, die unter industriell relevanten Reaktionsbedingungen signifikante Mengen der jeweiligen Carbonsäuren der eingesetzten Paraffine, im Falle der ODHE insbesondere Essigsäure, als Nebenprodukte bilden.

Gemäß Stand der Technik erfolgt die Reaktion bei der ODH vorzugsweise in Festbettreaktoren, insbesondere in gekühlten Rohrbündelreaktoren, z.B. mit Salzschmelzekühlung. Für stark exotherme Reaktionen, also insbesondere oxidative Reaktionen, wozu auch die ODH-E zählt, ist dabei allgemein die Verwendung eines Reaktorbettes mit mehreren Zonen bekannt. Grundlagen sind beispielsweise in der WO 2019/243480 A1 beschrieben. Diese Schrift offenbart dabei das Prinzip, dass verschiedene Katalysatorbetten bzw. entsprechende Reaktionszonen, die unterschiedliche Katalysatorbeladungen und/oder Katalysatoraktivitäten pro Raumeinheit aufweisen, zum Einsatz kommen.

Auch der Endbereich des entsprechenden (ein- oder mehrlagigen) Katalysatorbettes ist besonderen Belastungen ausgesetzt, da hier durch den Fortschritt der Reaktion zumeist nur noch eine geringe Restsauerstoffkonzentration enthalten ist. Hierbei soll unter dem Begriff "Ende" oder "Endbereich" der Bereich verstanden werden, in dem das durch den Reaktor oder ein entsprechendes Reaktionsrohr strömende Gas das Katalysatorbett verlässt. Die oben genannten Katalysatoren benötigen jedoch einen gewissen Mindestsauerstoffanteil im Reaktionsgas, um nicht zerstört zu werden. Auf der anderen Seite darf der Sauerstoffgehalt am Reaktoraustritt einen gewissen Grenzwert nicht überschreiten, um in folgenden Verfahrensschritten eine übermäßige Sauerstoffanreicherung und damit die mögliche Bildung einer explosionsfähigen Atmosphäre zu vermeiden.

Gemäß dem Stand der Technik ist die Verwendung einer nachgeschalteten Sauerstoffentfernung bekannt. Beispielsweise wird in der US 8,519,210 B2 die nachgeschaltete oder auch in einem ODH-E-Reaktor integrierte Sauerstoffentfernung beschrieben, wobei jedoch nur recht allgemein ein "oxygen elimination catalyst" erwähnt wird. Im beschreibenden Teil wird zur Sauerstoffelimination bzw. -enfernung eine Verbrennung von vorzugsweise Kohlenmonoxid und ggf. Kohlenwasserstoffen mit zwei und weniger Kohlenstoffatomen, was einen Ausbeuteverlust bedeutet, dargelegt. Gemäß dieser Schrift kann insbesondere ein vom eigentlichen ODH-E Katalysator unabhängiges und unterschiedliches Material zum Einsatz kommen und die zugrundeliegende Reaktion ist eine Umsetzung zu Kohlenmonoxid und/oder Kohlendioxid und Wasser.

Ein ähnliches Verfahren ist auch in WO 2017/144584 A1 beschrieben. Auch hier wird der Sauerstoffentfernungskatalysator bevorzugt im ODH-Reaktor stromab der Hauptreaktionszone eingesetzt, wobei der Sauerstoffentfernungskatalysator zwar ähnlich zum ODH-Katalysator ist, bevorzugt jedoch zusätzliche Elemente wie Sb, Pt, Pd und Cu oder Fe enthält, also bevorzugt eine andere Zusammensetzung aufweist oder sogar aus einer anderen Katalysatorklasse ausgewählt ist. Die genannten zusätzlichen Elemente katalysieren typischerweise ebenfalls zumeist die Umsetzung zu Kohlenmonoxid und/oder Kohlendioxid und Wasser.

Hauptanliegen einer weitestgehenden Sauerstoffentfernung im ODH-E Produktgas, wie z.B. in der US 8,519,210 B2 sowie WO 2017/144584 A1 ausgeführt, ist die Reduktion des Sauerstoffgehaltes, um dessen Anreicherung im Laufe der Aufarbeitung des Prozessgases im Trennteil und somit eine damit eventuell verbundene Bildung von zündfähigen Gemischen, z.B. in der Leichtgasfraktion einer sequentiellen kryogenen Trennung, zu vermeiden. Jedoch wird die Reduktion des Sauerstoffgehaltes, wie oben beschrieben, mit dem Nachteil einer teilweisen Wertproduktvernichtung erkauft.

Weiterhin kann der Sauerstoff in einem ODH-E Verfahren auch stromab des Reaktors an einer anderen Stelle, beispielsweise stromab einer Prozessgasverdichtung aber stromauf der kryogenen Trennung, über kupferbasierte Katalysatoren, wie beispielsweise in der WO 2018/153831 A1 beschrieben, erfolgen. Allerdings führt auch diese Variante zu einem gewissen Verlust von Ethan und dem Wertprodukt Ethylen, da auch hier der Sauerstoff katalytisch in Gegenwart und mit den anderen Prozessgaskomponenten (Ethan und Ethylen sowie Kohlenmonoxid) umgesetzt wird, wodurch eben ein Teil des Wertprodukts Ethylen durch Totaloxidation verloren wird.

Die vorliegende Erfindung stellt sich vor diesem Hintergrund die Aufgabe, ein verbessertes Verfahren zur Kontrolle und/oder Reduzierung des Sauerstoffgehalts am Ende eines zur ODH(-E) eingesetzten Reaktors bereitzustellen und auf diese Weise insbesondere eine erhöhte Katalysatorstandzeit sowie einen weitestgehend automatisierten Anlagenbetrieb zu ermöglichen, welcher die Anlagenüberwachung erleichtert und einen möglichst gleichmäßigen Anlagenbetrieb gewährleistet.

### Offenbarung der Erfindung

Die vorstehend genannte Aufgabe wird durch ein Verfahren und eine Anlage zur Herstellung eines Produktkohlenwasserstoffs mit den Merkmalen der jeweiligen unabhängigen Patentansprüche gelöst. Bevorzugte Ausgestaltungen der Erfindung sind jeweilis Gegenstand der abhängigen Patentansprüche und der nachfolgenden Beschreibung.

Vor der Erläuterung der Merkmale und Vorteile der vorliegenden Erfindung werden zunächst noch Grundlagen der Erfindung näher erläutert und bei der Beschreibung der Erfindung verwendete Begriffe definiert.

In Verfahren zur selektiven Oxidation von Kohlenwasserstoffen, insbesondere von Alkanen mit einem bis sechs Kohlenstoffatomen, besonders bei der oxidativen Dehydrierung von Alkanen mit einem bis sechs Kohlenstoffatomen und insbesondere bei der oxidativen Dehydrierung von Ethan zu Ethylen und Essigsäure über MoVNbOₓ-oder MoVNbTeOₓ-basierte Katalysatoren, ist zur Aufrechterhaltung der Katalysatorintegrität und -stabilität ein gewisser Mindestanteil an Sauerstoff im Produktgas bzw. im Reaktionsgas im Katalysatorbett notwendig, wie erwähnt. Ein zu geringer Sauerstoffanteil oder - im Extremfall - kein Sauerstoff im Produktgas, d.h. vorwiegend bzw. stark reduktive Bedingungen im Katalysatorbett, führen zu einer verstärkten Reduktion des Katalysatormaterials, was zum Verlust der aktiven kristallinen M1-Phase insbesondere eines MoVNbOₓ- oder MoVNbTeOₓ-Katalysatorsystems und somit zum Verlust an katalytischer Aktivität und ferner auch zum Verlust der mechanischen Integrität der primären Katalysatorkristalle und schließlich der Katalysatorformkörper führt.

Gemäß der WO 2017/144584 A1 können insbesondere reduktive Bedingungen, d.h. weitestgehende oder vollständige Sauerstofffreiheit im Produktgas, gerade bei MoVNbTeOₓ-Katalysatoren bei den für die ODH typischen Reaktionstemperaturen zu einem Tellurverlust aus dem Katalysator führen, was einerseits zum oben beschriebenen Aktivitäts-/Selektivitätsverlust des Katalysators führt sowie dessen mechanische Stabilität beeinträchtigt, und andererseits zu Problemen aufgrund von Tellurablagerungen in stromab befindlichen Anlagenteilen führen kann.

Da sich der Sauerstoff bei der Reaktion in Flussrichtung über das Katalysatorbett mit dem Ethan umsetzt, nimmt dessen Konzentration (bzw. dessen Partialdruck) im Reaktionsgas über die Katalysatorbettlänge (bzw. Reaktorlänge) ab und ist am Ende des Katalysatorbetts am geringsten, wie bereits erwähnt. Daher deaktiviert, im Falle einer zu geringen Sauerstoffkonzentration im Reaktions- bzw. Produktgas, das Katalysatorbett entgegen der Flussrichtung von hinten nach vorn, d.h. vom Katalysatorbettende nahe des Produktgasaustritt in Richtung stromauf zum Katalysatorbettanfang. Die Deaktivierungsrichtung des Katalysatorbetts ist also der Deaktivierungsrichtung einer typischen Katalysatorvergiftung, bei der das Katalysatorbett in Flussrichtung des Reaktionseinsatzes vom Katalysatorbettanfang her deaktiviert wird, entgegengesetzt.

Bestimmte Prozessstörungen, die den Sauerstoffgehalt am Reaktoraustritt bzw. am Ende des (ein- oder mehrlagigen) ODH-E-Katalysatorbettes übermäßig stark reduzieren, wirken sich also - wie oben erwähnt - auf die Katalysatoraktivität und Katalysatorselektivität in diesem Bereich aus, sofern nicht durch geeignete schnelle Maßnahmen eine Anpassung der Betriebsbedingungen erfolgt. Solche Prozessstörungen können z.B. (Aufzählung nicht abschließend) eine zu hohe Kühlmitteltemperatur oder eine zu geringe Sauerstoffkonzentration im Reaktionseinsatzstrom sein. Diese können z.B. durch Störungen in der Mengenregelung z.B. des Speisewassers, das zur Kühlung des Kühlmediums bereitgestellt wird, oder des Sauerstoff- oder Ethanflusses im Reaktionseinsatzstrom hervorgerufen werden. In der Realität zeigen sich dann als Folge solcher Prozessstörungen an dieser Stelle insbesondere bei niedrigen Sauerstoffkonzentrationen im Betrieb eines ODH-E Reaktors am Austritt eines (ein-oder mehrlagigen) Katalysatorbettes deutliche Fluktuationen in der Sauerstoffkonzentration. Damit korrespondiert häufig auch eine Temperaturschwankung in diesem Bereich und die Effekte schaukeln sich teilweise auch auf. Eine zu geringe Sauerstoffkonzentration in diesem Bereich kann also nicht nur den Katalysator schädigen, sondern auch einen unerwünschten instabilen Betrieb in der Austrittszone hervorrufen.

Hier ist eine wie in der US 8,519,210 B2 und der WO 2017/144584 A1 beschriebene Sauerstoffentfernung also nicht hilfreich, da als Gegenmaßnahme ein vergleichsweise hoher Sauerstoffgehalt am Ende des (ein- oder mehrlagigen) ODH-E-Katalysatorbettes zugelassen werden muss, was dann wieder zu einem entsprechenden erhöhten Verbrennungsanteil und entsprechendem Gesamtselektivitätsverlust in der nachfolgenden Sauerstoffentfernung führt.

Dieses Problem kann umgangen werden, indem die Leichtgasabtrennung im kryogenen Trennteil explosionsfest oder explosionsausbreitungshemmend, wie in der WO 2018/082945 A1 beschrieben, ausgeführt wird. Auf diese Weise kann im trockenen Prozessgas, d.h. nach einer Abtrennung des anfallenden Prozesskondensats (bestehend aus dem Reaktor zugeführten Wasser plus Reaktionswasser sowie Essigsäure und ggf. weiteren Oxygenatspuren), eine deutlich höhere Sauerstoffkonzentration von bis zu 2 Molprozent zugelassen werden.

### Merkmale und Vorteile der Erfindung

Die vorliegende Erfindung schlägt nun gegenüber dem eingangs erläuterten Stand der Technik einen abweichenden Ansatz vor, der insbesondere eine gezielte Einstellung des Sauerstoffgehalts am Reaktorende, hier als "Restsauerstoffgehalt" bezeichnet, umfasst. Erfindungsgemäß wird ein Restsauerstoffgehalt in einem Produkgemisch des eingesetzten Verfahrens zur Selektivoxidation vor und/oder nach einer Wasserentfernung hieraus in einem besonders bevorzugten Bereich, bzw. genauer oberhalb einer Minimalkonzentration sowie unterhalb einer Maximalkonzentration, gehalten, der bzw. die einerseits eine ausreichende Restmenge an Sauerstoff am Ende des oder der Katalysatorbetten sicherstellt und andererseits verhindert, dass stromab hiervon sicherheitskritische Werte erreicht werden.

Insgesamt schlägt die vorliegende Erfindung ein Verfahren zur Erzeugung eines Produktkohlenwasserstoffs vor, bei dem ein Einsatzkohlenwasserstoff in einem den Einsatzkohlenwasserstoff und Sauerstoff enthaltenden Einsatzgemisch unter Erhalt eines den Produktkohlenwasserstoff und Wasser enthaltenden Produktgemischs einer Selektivoxidation in einem Reaktor mit einem Katalysatorbett unterworfen wird. Das erfindungsgemäße Katalysatorbett kann dabei auch mehrere Zonen mit unterschiedlichen Katalysatoren, insbesondere mit unterschiedlicher Aktivität umfassen, die auch als separate Katalysatorbetten aufgefasst werden können. Ist daher nachfolgend von einem Katalysatorbett die Rede, kann dieses auch aus mehreren (Teil-)Betten oder entsprechenden Zonen ausgebildet sein. Bei dem Einsatzkohlenwasserstoff kann es sich insbesondere um Ethan oder ein anderes Paraffin mit bis zu sechs Kohlenstoffatomen handeln, bei dem Produktkohlenwasserstoff insbesondere um Ethylen oder ein anderes Olefin mit bis zu sechs Kohlenstoffatomen. Die Selektivoxidation wird insbesondere in der eingangs erläuterten und grundsätzlich bekannten Weise in Form einer oxidativen Dehydrierung mit den auch nachfolgend noch angegebenen Katalysatoren durchgeführt. Insbesondere kann die vorliegende Erfindung im Zusammenhang mit einer oxidativen Dehydrierung von Ethan zum Einsatz kommen und wird nachfolgend insbesondere unter Bezugnahme hierauf beschrieben. Ist nachfolgend von der Erzeugung eines Produktkohlenwasserstoffs im Singular die Rede, schließt dies nicht aus, dass auch andere Produktkohlenwasserstoffe und ein oder mehrere Koppel- oder Nebenprodukte wie insbesondere eine oder mehrere organische Säuren gebildet werden. Desgleichen schließt die Formulierung, wonach ein Einsatzkohlenwasserstoff verwendet wird, die Verwendung von weiteren Einsatzkohlenwasserstoffen nicht aus.

In dem erfindungsgemäßen Verfahren wird unter Abtrennung zumindest eines Teils des Wassers aus zumindest einem Teil des Produktgemischs ein Folgegemisch gebildet, und das Verfahren wird unter Einsatz der nachfolgend erläuterten Maßnahmen erfindungsgemäß derart durchgeführt, dass der in dem Einsatzgemisch enthaltene Sauerstoff bei der Selektivoxidation (nur) teilweise umgesetzt wird, so dass das Produktgemisch einen ersten Restsauerstoffgehalt und das Folgegemisch einen zweiten Restsauerstoffgehalt aufweist. Erfindungsgemäß beträgt der erste Restsauerstoffgehalt mindestens 0,41 Molprozent und der zweite Restsauerstoffgehalt mindestens 0,62 Molprozent, und es werden nachfolgend erläuterte Maßnahmen zur Einstellung des ersten und/oder zweiten Restsauerstoffgehalts ergriffen.

Die zumindest teilweise Wasserentfernung kann im Rahmen der vorliegenden Erfindung insbesondere durch kondensative Abscheidung von Wasser erfolgen, in welcher auch weitere Produkte der Selektivoxidation, im Falle der ODHE insbesondere Essigsäure, abgeschieden werden. Für das stromauf der entsprechenden Wasserentfernung vorliegende Produktgemisch wird nachfolgend auch der Begriff "feucht" verwendet, für das Produktgemisch oder dessen Teil, der zuvor der Entfernung von Wasser unterworfen wurde, der Begriff "trocken".

Erfindungsgemäß wird eine Erfassung des ersten und/oder zweiten Restsauerstoffgehalts unter Verwendung zumindest einer ersten Messeinrichtung vorgenommen. Ferner ist am Ende des Katalysatorbetts zumindest eine zweite Messeinrichtung bereitgestellt, die zur Erfassung einer eine Betriebsstabilität kennzeichnenden Größe eingerichtet ist, und unter Verwendung einer Prozesssteuerungs- und/oder Auswerteeinheit werden über einen Erfassungszeitraum Messdaten der wenigstens einen ersten Messeinrichtung und/oder der wenigstens einen zweiten Messeinrichtung erfasst und unter Erhalt von Folgedaten ausgewertet und/oder verarbeitet. Eine Verfahrenssteuerung wird dann in der weiter unten erläuterten Weise unter Verwendung der Folgedaten durchgeführt.

Gemäß einer Ausgestaltung der Erfindung wird das Verfahren insbesondere derart durchgeführt, dass der erste Restsauerstoffgehalt mindestens 0,41 Molprozent und der zweite Restsauerstoffgehalt mindestens 0,62 Molprozent beträgt. Das Verfahren kann dabei insbesondere derart durchgeführt werden, dass der zweite Restsauerstoffgehalt höchstens 2,0 Molprozent, höchstens 1,8 Molprozent oder insbesondere höchstens 1,5 Molprozent beträgt. Wie auch nachfolgend erläutert, können die genannten Werte und Zwischenwerte zur Definition von Schwellwerten verwendet werden, bei deren Überschreitung bestimmte betriebliche Maßnahmen ergriffen werden, die die entsprechende Einstellung bestimmter Betriebsparameter umfassen. Kern der Erfindung ist jedoch insbesondere die Erkenntnis, dass die Einstellung des Sauerstoffgehalts auf die genannten Wertebereiche an sich überraschende und unerwartete Vorteile bietet, wie insbesondere unter Bezug auf die beigefügten Beispiele belegt, wohingegen die zur Einstellung verwendeten Maßnahmen unterschiedlich sein können.

Die durch die vorliegende Erfindung realisierbaren Vorteile umfassen insbesondere eine Erhöhung der Laufzeit der Katalysatorschüttung, eine Erhöhung der Toleranz der Katalysatorschüttung gegenüber Störungen wie Abweichungen in Temperatur, Fluss, Zusammensetzung (insbesondere des Sauerstoffanteils), eine verbesserte Gewährleistung einer maximal akzeptablen Sauerstoffkonzentration am Reaktoraustritt, eine Vermeidung von instabilen Betriebszuständen und damit eine Erhöhung der Betriebssicherheit, ein frühzeitiges, automatisiertes Erkennen potenziell instabiler Betriebszustände und die Möglichkeit zur automatischen Einleitung von geeigneten Gegenmaßnahmen und dadurch weitgehende Automatisierung der Anlage, sowie die Möglichkeit der Erkennung lokaler instabiler Betriebszustände in einzelnen Reaktorbereichen und automatische Einleitung von geeigneten Gegenmaßnahmen.

Die vorliegende Erfindung sieht, wie erwähnt, vor, eine Erfassung des ersten und/oder zweiten Restsauerstoffgehalts unter Verwendung zumindest einer Messeinrichtung, hier lediglich der einfacheren Bezugnahme und nicht einschränkend als zumindest eine "erste" Messeinrichtung bezeichnet, vorzunehmen. Es können auch beispielsweise wenigstens eine Messeinrichtung zur Messung des ersten und wenigstens eine Messeinrichtung zur Erfassung des zweiten Restsauerstoffgehalts bereitgestellt sein. Bei beiden Messeinrichtungen handelt es sich um "erste" Messeinrichtungen im hier verwendeten Sprachgebrauch. Auch diese Ausgestaltung soll also durch die Angabe der "zumindest einen ersten" Messeinrichtung abgedeckt sein.

Die wenigstens eine erste Messeinrichtung ist vorteilhafterweise zur online-Erfassung der ersten und/oder zweiten Restsauerstoffkonzentration im feuchten und/oder trockenen Produktgemisch, vorzugsweise im Wesentlichen in Echtzeit, eingerichtet. Die Angabe "im Wesentlichen in Echtzeit" soll dabei eine Erfassung mit einer geringen Verzögerung darstellen, die maßgeblich durch die Zeit, die durch ein Totvolumen zwischen Probenahmestelle und einer Analyseneinheit hervorgerufen wird, definiert ist. Hierbei handelt es sich insbesondere um nicht mehr als 60 Sekunden, nicht mehr als 45 Sekunden, nicht mehr als 30 Sekunden, besonders bevorzugt nicht mehr als 15 Sekunden.Hierzu addiert sich die Ansprechzeit des entsprechenden Analysators, die bevorzugt nicht mehr als 10 Sekunden, besonders bevorzugt nicht mehr als 5 Sekunden beträgt.

Die Erfassung der Sauerstoffkonzentration kann z.B. über Gaschromatographie mittels eines Micro-Gaschromatographen, vorzugsweise aber über eine Messzelle, die sensitiv auf den Paramagnetismus des Sauerstoffmoleküls ist oder mittels einer Quantenkaskadenlaser-Messzelle erfasst werden. Die Erfassung mittels einer Messzelle, die sensitiv auf den Paramagnetismus des Sauerstoffmoleküls reagiert, oder mittels einer Quantenkaskadenlaser-Messzelle ist bevorzugt, da mit Hilfe dieser Methoden die Sauerstoffkonzentration mit sehr geringen Ansprechzeiten möglich ist. Mit anderen Worten ist die zumindest eine erste Messeinrichtung also zur gaschromatographischen und/oder paramagnetischen Sauerstoffmessung und/oder zur Sauerstoffmessung unter Verwendung eines Quantenkaskadenlasers eingerichtet.

Für die Selektivoxidation wird erfindungsgemäß, wie ebenfalls erwähnt und insoweit üblich, ein Reaktor mit einem Katalysatorbett verwendet. Unter dem Begriff des Katalysatorbetts wird hier eine Schüttung oder feste Struktur, die ein Katalysatormaterial und ggf. ein Inertmaterial als Träger aufweist, verstanden. Ist von mehreren Katalysatorbetten die Rede, können diese ohne oder mit zwischengeschalteten Zonen, insbesondere Inertzonen ohne Katalysatormaterial, in Flussrichtung hintereinander angeordnet sein. Generell entspricht die Flussrichtung eines Gases mit den umzusetzenden Reaktanden im vorliegend betrachteten Fall der Axialrichtung der in einem entsprechenden Reaktor verwendeten Reaktionsrohre.

Bevorzugt handelt es sich bei dem erfindungsgemäß eingesetzten Reaktor um einen Rohrbündelreaktor mit festen Katalysatorbetten im jeweils einzelnen Reaktionsrohr. Um die Gesamtwirtschaftlichkeit sowie eine erhöhte Betriebssicherheit zu erreichen, sind die einzelnen Reaktionsrohre dabei vorteilhafterweise mit mehreren Katalysatorbetten, insbesondere mit 1, 2, 3, 4, 5 Katalysatorbetten unterschiedlicher Aktivität und/oder Zusammensetzung, ausgestattet. Die Kühlung bzw. Beheizung erfolgt mit einem geeignetem Kühlmedium, insbesondere einem Thermoöl oder vorzugsweise einer Salzschmelze.

Am Ende des Katalysatorbetts - oder mehrerer Katalysatorbetten bei mehreren Reaktionsrohren - ist vorteilhafterweise zumindest eine zweite Messeinrichtung bereitgestellt, die zur Erfassung einer eine Betriebsstabilität kennzeichnenden Größe eingerichtet ist. Sind mehrere Reaktionszonen vorhanden, die als (Teil-)Betten eines Katalysatorbetts ausgebildet sind, befindet sich die zumindest eine zweite Messeinrichtung am Ende der in Strömungsrichtung "letzten" Zone bzw. des in Strömungsrichtung "letzten" (Teil-)Betts. Die Erfassung einer eine Betriebsstabilität kennzeichnenden Größe kann insbesondere zur Erkennung eines stabilen oder instabilen Betriebs vorgenommen werden, der sich aus den eingangs erwähnten Prozessstörungen ergibt. Insbesondere kann die zweite Messeinrichtung zur Erfassung einer Temperatur am Ende des Katalysatorbetts als der die Betriebsstabilität kennzeichnenden Größe eingerichtet sein.

Vorteilhafterweise wird im Rahmen der Erfindung auch ein Sauerstoffgehalt des Einsatzgemischs unter Verwendung einer Regelung vorgegeben. Der Sauerstoffgehalt kann dabei unter Verwendung zumindest einer dritten Messeinrichtung erfasst werden. Hierbei können insbesondere die zuvor erwähnten Messprinzipien verwendet werden. Der Sauerstoffgehalt des Einsatzgemischs kann aber alternativ oder zusätzlich auch rechnerisch aus den gemessenen Durchflussraten der zugeführten Einsatzströme erfasst werden.

Die vorliegende Erfindung sieht, wie erwähnt, vor, unter Verwendung einer Prozesssteuerungs- und/oder Auswerteeinheit über einen Erfassungszeitraum Messdaten der wenigstens einen ersten Messeinrichtung und/oder der wenigstens einen zweiten Messeinrichtung zu erfassen und unter Erhalt von Folgedaten auszuwerten und/oder zu verarbeiten.

Entsprechende Folgedaten können insbesondere einen über den Erfassungszeitraum ermittelten Wert des ersten und/oder des zweiten Restsauerstoffgehalts und/oder eine Schwankungsbreite hiervon und/oder einen über den Erfassungszeitraum ermittelten Wert der Betriebsstabilität kennzeichnenden Größe und/oder eine Schwankungsbreite hiervon umfassen. Anders ausgedrückt könnendie Messdaten der entsprechenden Sauerstofferfassung so verarbeitet werden, dass kontinuierlich (i) die zeitlich gemittelte Sauerstoffkonzentration im trockenen und/oder feuchten Produktgemisch sowie (ii) die absolute zeitliche Schwankungsbreite der Sauerstoffkonzentration im trockenen und/oder feuchten Produktgemisch bestimmt und ausgegeben werden. Die Messdaten der vorteilhafterweise verwendeten Temperaturerfassung am Ende des Katalysatorbetts können so verarbeitet werden, dass die zeitlich gemittelte Temperatur sowie (ii) die absolute zeitliche Schwankungsbreite der Temperatur bestimmt und ausgegeben werden.

Generell können für die zeitliche statistische Auswertung unterschiedlich lange Zeitintervalle herangezogen und auch gleichzeitig ausgewertet werden, z.B. (nicht ausschließend) ein gleitender Mittelwert über kurze und etwas längere Zeitskalen. Stellvertretend für die Quantifizierung der Schwankungen wird hier der Abstand zwischen Minimalwert und Maximalwert innerhalb eines betrachteten Zeitintervalls verwendet. Alternativ kann auch jede andere Form einer Abweichungsbestimmung herangezogen werden, wie z.B. Standardabweichung, Standardabweichung vom Mittelwert oder ähnlich geläufige Größen innerhalb der Statistik.

Auf Grundlage der Folgedaten, insbesondere zumindest eines der erwähnten Werte, kann sodann die erwähnte Verfahrenssteuerung durchgeführt werden. Diese umfasst insbesondere die Anpassung wenigstens eines Betriebsparameters, wie nachfolgend in zwei auch miteinander kombinierbaren Ausgestaltungen erläutert.

Der für die Selektivoxidation verwendete Reaktor wird insbesondere unter Verwendung wenigstens eines Kühlmittels gekühlt, und der wenigstens eine Betriebsparameter kann in einer entsprechenden Ausgestaltung eine Kühlmitteltemperatur des wenigstens einen Kühlmittels bzw. Kühlmedium darstellen oder umfassen.

Das Kühlmittel kann dabei im Gleich- oder im Gegenstrom zu dem durch den Reaktor strömenden Prozessgas führen. Insbesondere bei einer Führung des Kühlmittels, insbesondere einer Salzschmelze, im Gegenstrom zu dem durch den Reaktor strömenden Prozessgas lässt sich ein zusätzlicher Vorteil erzielen, da hier die Reaktionswärme aus den stromab (d.h. in Richtung Reaktorausgang) einer Reaktionszone liegenden Reaktionszonen in den stromauf liegenden Reaktionszonen teilweise mit ausgenutzt werden kann. Ebenso können im Rahmen der vorliegenden Erfindung unterschiedliche Kühlkreisläufe in Kombination mit unterschiedlichen Katalysatorlagen verwendet werden (wie auch in der WO 2019/243480 A1 aufgeführt).

Die Kühlmitteltemperatur kann insbesondere automatisch durch eine programmtechnische Routine insbesondere schrittweise reduziert werden, sofern die mittlere Sauerstoffkonzentration im trockenen und/oder feuchten Produktgemisch kleiner als 0,62 Molprozent oder kleiner als 0,41 Molprozent ist. Als zusätzliche oder alternative Bedingung kann auch vorgesehen sein, dass die Schwankungsbreite der Sauerstoffkonzentration im trockenen und/oder feuchten Produktgemisch größer als 0,2 Prozentpunkte oder größer als 0,13 Prozentpunkte und/oder die Schwankungsbreite der Temperatur am Katalysatorbettende größer als 0,4 K ist. Die Reduktion der Kühlmitteltemperatur kann insbesondere so lange erfolgen, bis die mittlere Sauerstoffkonzentration im trockenen und/oder feuchten Produktgemisch mindestens 0,62 Molprozent oder mindestens 0,41 Molprozent beträgt. Als zusätzliche oder alternative Bedingung kann auch vorgesehen sein, dass die maximale Schwankungsbreite der Sauerstoffkonzentration im trockenen und/oder feuchten Produktgemisch kleiner als 0,2 Prozentpunkte oder kleiner als 0,13 Prozentpunkte und/oder die Schwankungsbreite der Temperatur am Katalysatorbettende kleiner als 0,4 K ist. Eine derartige Ausgestaltung ist insbesondere dann bevorzugt, wenn der Umsatz zum Erreichen des stabilen Zustandes nahezu konstant bleiben soll und eine längere Zeit bis zum Erreichen eines stabilen Betriebs akzeptiert werden kann.

Der wenigstens eine Betriebsparameter kann jedoch auch einen Sauerstoffgehalt des Einsatzgemischs darstellen oder umfassen.

Die Sauerstoffkonzentration bzw. das molare Verhältnis zwischen Sauerstoff und dem Eduktkohlenwasserstoff, insbesondere Ethan, kann dann im Reaktionseinsatzstrom bzw. Einsatzgemisch insbesondere automatisch durch eine programmtechnische Routine und insbesondere schrittweise erhöht werden, sofern die mittlere Sauerstoffkonzentration im trockenen und/oder feuchten Produktgemisch kleiner als 0,62 Molprozent oder kleiner als 0,41 Molprozent ist. Als zusätzliche oder alternative Bedingung kann auch vorgesehen sein, dass die Schwankungsbreite der Sauerstoffkonzentration im trockenen und/oder feuchten Produktgemisch größer als 0,2 Prozentpunkte oder größer als 0,13 Prozentpunkte ist und/oder die Schwankungsbreite der Temperatur am Katalysatorbettende größer als 0,4 K ist. Die Erhöhung der Sauerstoffkonzentration bzw. des molaren Verhältnisses zwischen Sauerstoff und dem Eduktkohlenwasserstoff, insbesondere Ethan, im Reaktionseinsatzstrom bzw. Einsatzgemisch kann insbesondere so lange erfolgen, bis die mittlere Sauerstoffkonzentration im trockenen und/oder feuchten Produktgemisch mindestens 0,62 Molprozent oder mindestens 0,41 Molprozent beträgt. Als zusätzliche oder alternative Bedingung kann auch vorgesehen sein, dass die maximale Schwankungsbreite der Sauerstoffkonzentration im trockenen und/oder feuchten Produktgemisch kleiner als 0,2 Prozentpunkte oder kleiner als 0,13 Prozentpunkte ist und/oder die Schwankungsbreite der Temperatur am Katalysatorbettende kleiner als 0,4 K ist. Diese Ausgestaltung ist insbesondere dann bevorzugt, wenn eine rasche Einstellung eines stabilen Betriebszustandes erforderlich ist und wenn insbesondere gleichzeitig der Kohlenwasserstoffumsatz (Ethanumsatz) gesteigert werden soll.

Die genaue Wirkweise der beschriebenen, insbesondere programmtechnisch realisierten, Routinen kann u.a. auch unter Berücksichtigung verschiedener zeitlicher Auswertungsintervalle variiert werden, um auf diese Weise beispielsweise spezifischen Reaktorgestalungen angepasst werden zu können.

Im Gegensatz zu einem Pilotreaktor mit einem Reaktionsrohr, wie er in den im Zusammenhang mit den Figuren erläuterten Beispielen eingesetzt wird, weisen kommerzielle Reaktoren eine große Anzahl an Reaktionsrohren (mehrere 1.000 bis mehrere 10.000) auf. Die Reaktionsführung in den verschiedenen Reaktionsrohren wird zwangsläufig einer Schwankungsbreite unterworfen sein, z.B. aufgrund von unterschiedlichen Strömungsbedingungen auf der Kühlmittelseite oder auch variierenden Mischungsbedingungen auf der Prozessgasseite.

Eine instrumentenbasierte Überwachung jedes einzelnen Reaktionsrohrs ist praktisch nicht möglich. Insbesondere kann die Prozessgaszusammensetzung am Reaktoraustritt nur für das resultierende Gesamtgemisch gemessen werden. Wie oben beschrieben, kann eine erhöhte Schwankungsbreite dieser Messgröße, z.B. des Sauerstoffs am Reaktoraustritt, nur auf einen global instabilen Reaktorbetrieb hindeuten, da bei der Überwachung des Prozessgases zwangsläufig eine Mittelung über alle Reaktionsrohre entsteht, und somit erst zu einem fortgeschrittenem Eskalationsstadium in die Prozesssteuerung einfließen.

Besonders geeignet für die frühzeitige Detektion von instabilen Reaktionsbedingungen in einzelnen Reaktorbereichen ist hingegen die Analyse von Temperaturmessungen in Reaktionsrohren. In der industriellen Praxis ist es gebräuchlich, eine begrenzte Anzahl von Reaktionsrohren mit Temperaturfühlern entlang des Katalysatorbetts auszustatten. Mit diesen entsprechenden einzelnen Messstellen können gewissermaßen repräsentative Stichproben der Temperaturbedingungen in unterschiedlichen Bereichen des Reaktors vorgenommen werden.

Ein wesentliches Merkmal einer besonders bevorzugten Ausgestaltung der vorliegenden Erfindung besteht darin, dass die Überprüfung der zeitlich gemittelten Temperatur und deren zeitliche Schwankungsbreite für mehrere Reaktionsrohre erfolgt, bevorzugt für alle Reaktionsrohre, die mit Einrichtungen zur Temperaturmessung im Katalysatorbett (insbesondere am Katalysatorbettende) ausgestattet sind.

Die programmtechnische Anpassung der Reaktionsführung, d.h. die Anpassung der Betriebsparameter, kann beispielsweise erfolgen, wenn Daten von mindestens von einer, zwei, fünf, oder zehn oder mindestens 1, 2, 5 oder 10 Prozent der Temperaturmessstellen bzw. zweiten Messeinrichtungen auf einen instabilen Reaktionsbetrieb in zumindest einzelnen Bereichen des Reaktors hindeuten (auf Basis der oben genannten Kriterien).

In allgemeinerer Formulierung kann der für die Selektivoxidation verwendete Reaktor also eine Vielzahl von Reaktionsrohren aufweisen, wobei die wenigstens eine erste Messeinrichtung und/oder die wenigstens eine zweite Messeinrichtung einem oder einem Teil der Reaktionsrohre zugeordnet ist.

Wie mehrfach erwähnt, kann die vorliegende Erfindung insbesondere in einem Verfahren zur oxidativen Dehydrierung von Ethan eingesetzt werden, in welchem Fall der Produktkohlenwasserstoff Ethylen und der Einsatzkohlenwasserstoff Ethan ist, und in welchem Fall dem die Selektivoxidation als oxidative Dehyrierung unter Verwendung eines zumindest die Elemente Molybdän, Vanadium, Niob und optional Tellur enthaltenden Katalysators, insbesondere eines Mischoxids der genannten Elemente, durchgeführt wird.

Die oxidative Dehydrierung kann insbesondere bei einer Temperatur des Katalysators in einem Bereich zwischen 240 und 500°C, bevorzugt zwischen 280 und 450 °C, weiter vorzugsweise zwischen 300 und 400 °C durchgeführt w erden. Für die oxidative Dehydrierung können insbesondere ein oder mehrere Reaktoren verwendet werden, wobei ein Gesamtdruck des Reaktionseinsatzstromes bzw. Einsatzgemischs am Eintritt des oder der Reaktoren in einem Bereich zwischen 1 und 10 bar (abs.), bevorzugt zwischen 2 und 6 bar (abs.) bereitgestellt werden kann. Das Einsatzgemisch kann insbesondere mit einem Wasseranteil bereitgestellt werden, der zwischen 5 und 95 Volumenprozent, insbesondere zwischen 10 und 50 Volumenprozent, weiter insbesondere zwischen 14 und 35 Volumenprozent eingestellt werden kann. In einer besonders bevorzugten Ausgestaltung wird eine ODHE eingesetzt, bei der das molare Verhältnis von Wasser zu Ethan im Reaktionseinsatzstrom mindestens 0,23 beträgt.

Eine Anlage zur Erzeugung eines Produktkohlenwasserstoffs, die dafür eingerichtet ist, einen Einsatzkohlenwasserstoff in einem den Einsatzkohlenwasserstoff und Sauerstoff enthaltenden Einsatzgemisch unter Erhalt eines den Produktkohlenwasserstoff und Wasser enthaltenden Produktgemischs einer Selektivoxidation zu unterwerfen, und die dafür eingerichtet ist, unter Abtrennung zumindest eines Teils des Wassers aus zumindest einem Teil des Produktgemischs ein Folgegemisch zu bilden, wobei die Anlage dafür eingerichtet ist, das Verfahren derart durchzuführen, dass der in dem Einsatzgemisch enthaltene Sauerstoff bei der Selektivoxidation teilweise umgesetzt wird, so dass das Produktgemisch einen ersten Restsauerstoffgehalt und das Folgegemisch einen zweiten Restsauerstoffgehalt aufweist, ist ebenfalls Gegenstand der vorliegenden Erfindung. Hierbei beträgt der erste Restsauerstoffgehalt mindestens 0,41 Molprozent und der zweite Restsauerstoffgehalt mindestens 0,62 Molprozent.

Zu Ausgestaltungen einer entsprechenden Anlage, die insbesondere zur Durchführung eines Verfahrens oder einer Ausgestaltung wie zuvor erläutert eingerichtet sein kann, sei auf die obigen Erläuterungen ausdrücklich verwiesen.

Die Erfindung wird nachfolgend unter Bezugnahme spezifischer erfindungsgemäßer Beispiele mit zugehörigen Figuren weiter erläutert.

### Ausführungsbeispiele

Der zuvor angeführte instabile Betrieb im Falle einer zu geringen Konzentration an Sauerstoff im (trockenen) Produktgemisch wurde in Versuchen mit Hilfe eines Pilotreaktors untersucht. Der verwendete Pilotreaktor ist ein durch Salzschmelze gekühlter Festbettreaktor. Es handelt sich dabei um den gleichen Pilotreaktor, mit dem die in der WO 2019/243480A1 beschriebenen Ergebnisse erzielt wurden. Der Pilotreaktor ist als ein Rohr-in-Rohr Reaktor ausgeführt, wobei das Innenrohr mit dem Katalysator-Festbett befüllt ist (Reaktionsraum). Zwischen der Wandung des Reaktionsraumes und dem Außenrohr befindet sich der Kühlmittelraum, d.h. dieser Raum wird mit dem Kühlmittel, hier mit einer flüssigen Salzschmelze, im Gegenstrom zur Flussrichtung des Reaktionseinsatzstromes durchströmt. Bei der Salzschmelze handelt es sich um eine Mischung aus Natriumnitrit, Natriumnitrat sowie Kaliumnitrat. Die Dimensionen (d.h. Länge, Innendurchmesser und Wandstärke) des Reaktionsraumes des Pilotreaktors entsprechen den typischen Dimensionen eines einzelnen Rohres aus einem typischen kommerziellen (großtechnischen) Rohrbündelreaktor. Somit ist der Pilotreaktor als ein echtes Abbild einer großtechnischen Anlage anzusehen, da sich in diesem Pilotreaktor aufgrund seiner Geometrie die gleichen Verhältnisse (Strömungsfeld, Temperatur-bzw. Temperaturgradienten, Druckgradienten etc.) wie in einem einzelnen Rohr eines technischen Rohrbündelreaktors einstellen und sich somit die Reaktion unter realen technischen Bedingungen testen lassen.

Für den Testbetrieb wurde der Pilotreaktor mit einem hinsichtlich der katalytischen Aktivität dreistufigen Katalysatorbett gefüllt. Dabei war das katalytisch aktive Grundmaterial für jede Stufe exakt das gleiche. Das Bett war dabei so angeordnet, dass die katalytische Aktivität in Flussrichtung des Reaktionseinsatzstromes zunahm. Die unterschiedliche Aktivitätsabstufung wurde (wie auch in der WO 2019/243480 A1 beschrieben) durch Katalysatorformkörper (Ringe) mit unterschiedlich hoher Beimengung an Binder, der zur Formung der Formkörper benötigt wird, zum exakt gleichen katalytisch aktiven Grundmaterial erreicht. Der Binder fungiert also gleichzeitig auch als Verdünnungsmittel des aktiven Katalysatormaterials. Jede Katalysatorschicht wies die gleiche Höhe und somit das gleiche Volumen auf. Die Masse an eingefülltem Katalysatoraktivmaterial, d.h. die Masse der reinen Aktivkomponente des Katalysators ohne die jeweiligen Binderanteile betrug 2,13 kg. Stromauf und stromab des dreistufigen Katalysatorbetts befand sich jeweils eine Schüttung aus Inertmaterial gleicher Form und ähnlicher Größe wie die Katalysatorformkörper.

Der Pilotreaktor wurde sodann über einen Zeitraum von ca. 1700 h (ca. 71 Tage) mit einem Reaktionseinsatzstrom im Wesentlichen bestehend aus Ethan, Sauerstoff sowie Wasser(dampf) betrieben. Das molare Verhältnis von Ethan zu Sauerstoff zu Wasser(dampf) im Reaktionseinsatzstrom betrug 59 zu 24 zu 17. Der Druck am Reaktoreintritt betrug 3,81 bara, die GHSV betrug 1088 h⁻¹ (bzw. Normkubikmeter Gas pro Stunde und pro Kubikmeter Katalysator). Über den gesamten Testzeitraum war eine gleichbleibende, stabile und sehr gute Reaktor- bzw. Katalysatorperformance hinsichtlich Ethanumsatz und Selektivitäten zu den gewünschten kommerziellen Wertprodukten Ethylen und Essigsäure zu beobachten. Der Ethanumsatz lag bei ca. 52,5%, die Selektivität zu Ethylen bei ca. 82,5% und die Selektivität zu Essigsäure bei ca. 12%, d.h. es konnte eine Gesamtselektivität zu kommerziellen Wertprodukten von mehr als 94% erzielt werden.

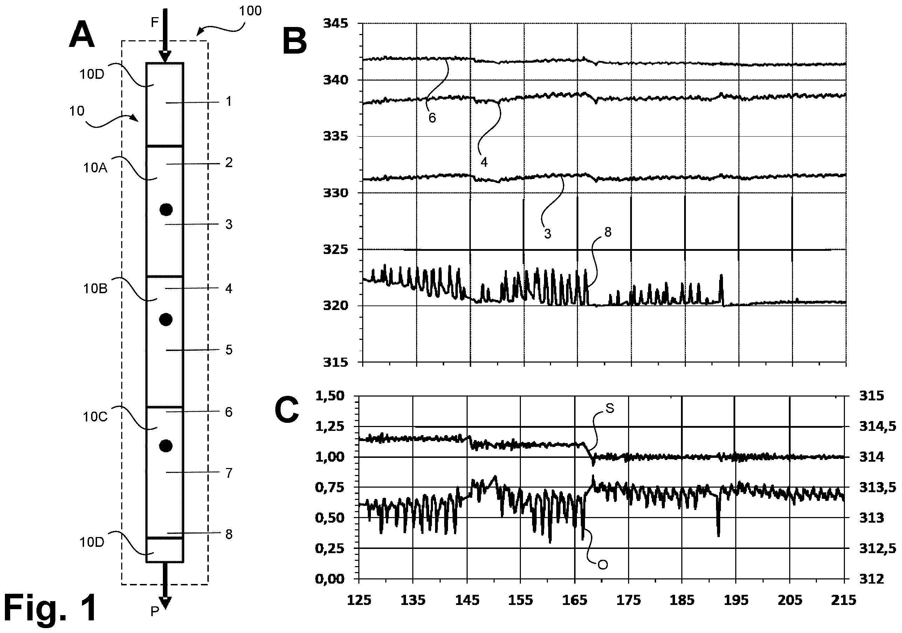

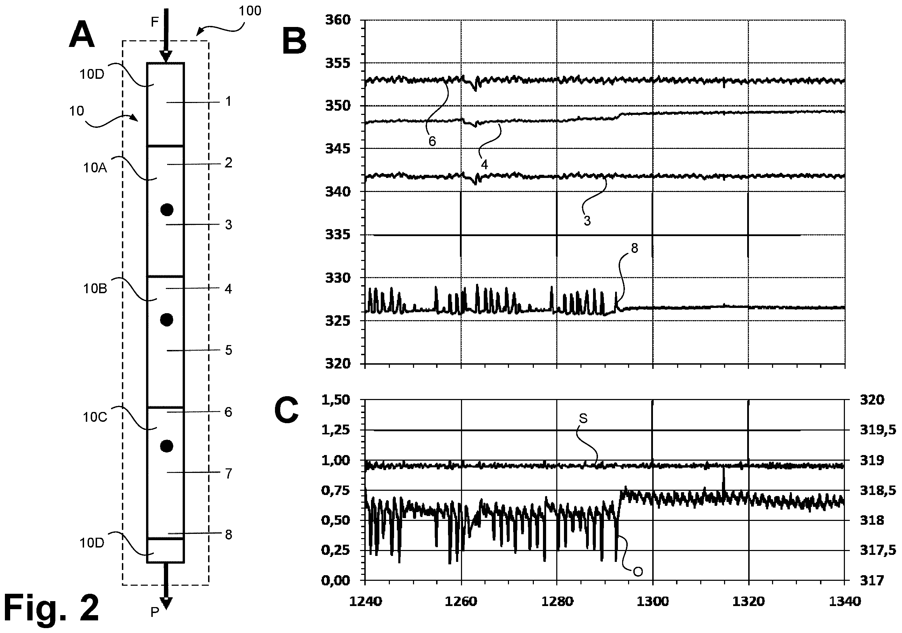

Die Sensitivität der Stabilität des Reaktionsbetriebs in Abhängigkeit von der mittleren Sauerstoffkonzentration im trockenen Produktgas zeigen dabei die Figuren 1 und 2 sowie die zugehörigen Tabellen 1 und 2.

### Beispiel 1

Beim Betrieb eines ODHE-Reaktors kann, wie erwähnt, eine erhöhteKühlmitteltemperatur als Prozessstörung auftreten. Eine nur leicht erhöhte Kühlmitteltemperatur im Bereich von wenigen zehntel Kelvin hat keinen signifikanten Einfluss auf den Ethanumsatz, jedoch einen deutlichen Einfluss auf die Sauerstoffkonzentration im trockenen Produktgas.

Es kann aber auch beobachtet werden, dass nach einer anfänglichen Einlaufzeit des Katalysators und Betrieb des Reaktors auf einem für das Verfahren angestrebten Umsatzpunkt, die katalytische Aktivität weiterhin über einen gewissen Zeitraum kontinuierlich (sehr langsam) zunimmt, dies jedoch in einem Rahmen erfolgt, dass diese Zunahme keinen signifikanten Effekt auf den Ethanumsatz, jedoch einen deutlichen Effekt auf die Sauerstoffkonzentration im trockenen Produktgas hat. Eine schleichende Zunahme der Katalysatoraktivität ist zwar keine Prozessstörung im engeren Sinne, die Auswirkungen auf die Stabilität des Betriebs sind jedoch gleich.

Dies wird anhand der Figur 1 und der Tabelle 1 beschrieben.

Im linken Teil A der Figur 1 ist dabei ein Reaktionsrohr 10 mit drei Katalysatorfüllungen bzw. Katalysatorbetten 10A, 10B, 10C, deren Aktivität in Richtung des Reaktoraustritts, der in Figur 1 unten dargestellt ist, zunimmt. Nicht mit Katalysator ausgestattete Bereiche sind mit 10D angegeben. Ein Reaktor 100, dessen Teil das Reaktionsrohr 10 ist, ist stark vereinfacht angedeutet. Dem Reaktionsrohr 10 wird ein Einsatzgemisch F zugeführt und ein (noch feuchtes) Produktgemisch P entnommen, aus dem der Großteil des enthaltenen Wassers sowie der enstandenen Essigsäure weiter stromab, vor der ersten Messeinrichtung (online Sauerstoffmessung mittels einer paramagnetischen Messzelle) auskondensiert werden. In den Katalysatorbetten sind jeweils ungefähre Positionen von Temperaturhotspots mit ausgefüllten Kreisen dargestellt. Temperaturmessstellen sind mit 1 bis 8 veranschaulicht. Messstelle 8 befindet sich dabei ca. 5 cm vor dem Ende des Katalysatorbetts 10C in Richtung Reaktoraustritt.

In Diagrammen B und C sind, jeweils gegenüber einer Laufzeit ("Time on Stream") auf der Horizontalachse, einerseits eine Katalysatortemperatur an den Messstellen 3, 4, 6 und 8 auf der Vertikalachsein °C (Diagramm B) und a ndererseits eine Temperatur einer Salzschmelze S auf der rechten Vertikalachse in °C sowie ein Restsauerstoffgehalt des trockenen Produktgemischs auf der linken Vertikalachse in Volumen- bzw. Molprozent (Diagramm C) dargestellt (wie oben erwähnt befindet sich die Sauerstoffmessstelle in dieser Pilotanlage stromab der Abtrennung von Prozesskondensat, d.h. im trockenen Prozessgas). Die Tabelle 1 gibt nochmals diese und entsprechende weitere Werte in entsprechenden Zeitintervallen an.

**Tabelle 1**

| **Größe** | **Einheit** | **125 bis 145 Stunden** | **145 bis 167 Stunden** | **167 bis 215 Stunden** |
|---|---|---|---|---|
| Salztemperatur | °C | 314,3 | 314,2 | 314,0 |
| Temperaturschwankungen an Messstelle 8 | K | von 0,6 auf 2,7 steigend | 3,0 | 1,4 bis kleiner 0,4 |
| Mittlere Sauerstoffkonzentration im trockenen Produktemisch, gemessen | mol% | 0,57 | 0,61 | 0,70 |
| Mittlere Sauerstoffkonzentration im feuchten Produkgemisch, berechnet | mol% | 0,38 | 0,40 | 0,46 |
| Maximale Schwankung der Sauerstoffkonzentration im trockenen Produkgemisch, gemessen | mol% | 0,27 | 0,4 | 0,15 bis 0,06 |
| Maximale Schwankung der Sauerstoffkonzentration im feuchten Produktgemisch, berechnet | mol% | 0,18 | 0,26 | 0,10 bis 0,04 |
| Umsatz Ethan | % | 52,5 | 52,4 | 52,3 |

Die Berechnung der maximale Schwankung der Sauerstoffkonzentration im feuchten Produktgemisch erfolgte derart, dass für die maximalen Schwankungen im feuchten Produktgemisch die gleiche Relation der maximalen Schwankung zum Mittelwert wie für das trockene Produktgemisch angenommen wurde.

Wie aus Figur 1 und Tabelle 1 ersichtlich, werden bei einer mittleren Kühlmitteltemperatur bzw. Salzschmelzetempeartur von 314,3 °C (Zeitraum 125 bis 145 Stunden in Figur 1) periodische Schwankungen der Temperatur am Ende des Katalysatorbetts (Messstelle 8) sowie der Sauerstoffkonzentration im trockenen Produkgemisch beobachtet. Dabei wies die Sauerstoffkonzentration ein Minimum auf, wenn die Temperatur an der Messstelle 8 ein Maximum aufwies und umgekehrt. Diese Schwankungen waren bei der Kühlmitteltemperatur von 314,3 °C nicht konstant, sondern nahmen an Intensität zu (max. Temperaturschwankungen stiegen von etwa 0,6 auf 2,7 K, max. Schwankungen der Sauerstoffkonzentration stiegen auf 0,27 mol%). Das heißt, das System ist instabil und schaukelte sich langsam auf. Daraufhin wurde die Kühlmitteltemperatur um 0,1 K gesenkt (Zeitraum 145 bis 167 Stunden in Figur 1), was offensichtlich zu einer Stabilisierung der Temperatur- sowie Sauerstoffschwankungen sowie zu einem geringfügigen Anstieg in der Sauerstoffkonzentration des trockenen Produkgemisch führte. Jedoch waren die maximalen Schwankungen der Temperatur am Katalysatorbettende mit 3,0 K bzw. maximalen Schwankungen der Sauerstoffkonzentration mit 0,4 mol% gegenüber den beobachteten Schwankungen im Zeitraum 125 bis 145 Stunden größer. Eine weitere Verringerung der Kühlmitteltemperatur um 0,2 K auf 314,0 °C führte zunächst zu einer deutlichen Verringerung der Temperaturschwankungen am Katalysatorbettende auf zunächst 1,4 K. Nach einer Laufzeit von ca. 20 h bei diesen Bedingungen waren die Temperaturschwankungen kleiner als 0,4 K. Es sei hier erwähnt, dass alle anderen Temperaturmessstellen während der gesamten Zeit gleichbleibende sehr geringe Schwankungen aufwiesen und von den hier beschriebenen Maßnahmen nicht beeinflusst wurden. Der Effekt tritt also tatsächlich nur am Katalysatorbettende auf. Im gleichen Zeitraum (167 bis 215 Stunden) stieg aufgrund der etwas geringeren Kühlmitteltemperatur die mittlere Sauerstoffkonzentration im trockenen Produktgas auf 0,70% mit maximalen Schwankungen von zunächst max. 0,15 mol% und nach ca. 20 h Laufzeit auf dem Betriebspunkt von nur noch 0,06 mol%. Der Ethanumsatz wurde durch die leichte Kühlmitteltemperaturverringerung dabei praktisch nicht beeinflusst und blieb während der ganzen Zeit auf nahezu dem gleichen Niveau von 52,3 bis 52,5%. Eine geringfügige Absenkung der Kühlmitteltemperatur führt also zu einer deutlichen Stabilisierung des Reaktionsverhaltens bei Beibehaltung des angestrebten Ethanumsatzes.

### Beispiel 2

Zur Beschreibung der Figur 2 wird bezüglich der dargestellten Parameter auf die Beschreibung von Figur 1 verwiesen.

**Tabelle 2**

| **Größe** | **Einheit** | **1240 bis 1290 Stunden** | **1290 bis 1340 Stunden** |
|---|---|---|---|
| Salztemperatur | °C | 318,9 | 318,9 |
| Verhältnis von Sauerstoff zu Ethan im Einsatzgemisch und relative Änderung gegenüber Zeitintervall 1240 bis 1290 Stunden (in Klammern) | mol/mol (%) | 0,408 | 0,417 (+2,2) |
| Temperaturschwankungen an Messstelle 8 | K | Max. 2,8 | < 0,2 |
| Mittlere Sauerstoffkonzentration im trockenen Produktgemisch, gemessen | mol% | 0,54 | 0,65 |
| Mittlere Sauerstoffkonzentration im feuchten Produktgemisch, berechnet | mol% | 0,35 | 0,43 |
| Maximale Schwankung der Sauerstoffkonzentration im trockenen Produktgemisch, gemessen | mol% | 0,62 | 0,15 |
| Maximale Schwankung der Sauerstoffkonzentration im feuchten Produktgemisch, berechnet | mol% | 0,41 | 0,10 |

Bei gleicher Kühlmitteltemperatur führte die geringfügige Erhöhung des molaren Verhältnisses von Sauerstoff zu Ethan im Einsatzgemisch um 2,2% von 0,408 auf 0,417 zu einer Abnahme der Temperaturschwankungen von 2,8 K am Katalysatorbettende auf unter 0,2 K. Auch hier bleiben die sehr geringen Schwankungen der übrigen Temperaturen im Katalysatorbett von der Erhöhung der Sauerstoffkonzentration im Einsatzgemisch unbeeinflusst. Die Erhöhung der Sauerstoffkonzentration im Einsatzgemisch führte zu einer Erhöhung der mittleren Sauerstoffkonzentration im trockenen Produktgemisch von 0,54 mol% auf 0,65 mol%. Gleichzeitig nahmen die maximalen (absoluten) Schwankungen der Sauerstoffkonzentration im trockenen Produktgasstrom von 0,62 auf 0,15 mol% ab. Die Erhöhung der Sauerstoffkonzentration im Einsatzgemisch führte, anders als bei Beispiel 1, zu einer Erhöhung des Ethanumsatzes um 0,9 Prozentpunkte von 51,8% auf 52,7%. Durch diese Anpassung bzw. Deeskalationsmethode kann also außerdem auch der Ethanumsatz in gewissem Maße gesteigert werden.

## Patentansprüche

1. Verfahren zur Erzeugung eines Produktkohlenwasserstoffs, bei dem ein Einsatzkohlenwasserstoff in einem den Einsatzkohlenwasserstoff und Sauerstoff enthaltenden Einsatzgemisch unter Erhalt eines den Produktkohlenwasserstoff und Wasser enthaltenden Produktgemischs einer Selektivoxidation in einem Reaktor mit einem Katalysatorbett unterworfen wird, und bei dem unter Abtrennung zumindest eines Teils des Wassers aus zumindest einem Teil des Produktgemischs ein Folgegemisch gebildet wird, wobei das Verfahren derart durchgeführt wird, dass der in dem Einsatzgemisch enthaltene Sauerstoff bei der Selektivoxidation teilweise umgesetzt wird, so dass das Produktgemisch einen ersten Restsauerstoffgehalt und das Folgegemisch einen zweiten Restsauerstoffgehalt aufweist, **dadurch gekennzeichnet, dass** eine Erfassung des ersten und/oder des zweiten Restsauerstoffgehalts unter Verwendung zumindest einer ersten Messeinrichtung vorgenommen wird, dass am Ende des Katalysatorbetts zumindest eine zweite Messeinrichtung bereitgestellt ist, die zur Erfassung einer eine Betriebsstabilität kennzeichnenden Größe eingerichtet ist, dass unter Verwendung einer Prozesssteuerungs- und/oder Auswerteeinheit über einen Erfassungszeitraum Messdaten der wenigstens einen ersten Messeinrichtung und/oder der wenigstens einen zweiten Messeinrichtung erfasst und unter Erhalt von Folgedaten ausgewert und/oder verarbeitet werden, und dass auf Grundlage der Folgedaten eine Verfahrenssteuerung durchgeführt wird.

2. Verfahren nach Anspruch 1, das umfasst, dass der erste Restsauerstoffgehalt mindestens 0,41 Molprozent und der zweite Restsauerstoffgehalt mindestens 0,62 Molprozent beträgt.

3. Verfahren nach Anspruch 1 oder Anspruch 2, bei dem der erste Restsauerstoffgehalt mindestens 0,41 Molprozent und der zweite Restsauerstoffgehalt mindestens 0,62 Molprozent beträgt, und/oder bei dem der zweite Restsauerstoffgehalt höchstens 2, Molprozent, höchstens 1,8 Molprozent oder höchstens 1,5 Molprozent beträgt.

4. Verfahren nach Anspruch 3, bei dem die zumindest eine erste Messeinrichtung zur gaschromatographischen und/oder paramagnetischen Sauerstoffmessung eingerichtet ist und/oder zur Sauerstoffmessung unter Verwendung eines Quantenkaskadenlasers eingerichtet ist.

5. Verfahren nach Anspruch 1, bei dem die zumindest eine zweite Messeinrichtung zur Erfassung einer Temperatur am Ende des Katalysatorbetts als der die Betriebsstabilität kennzeichnenden Größe eingerichtet ist.

6. Verfahren nach Anspruch 5, bei dem ein Sauerstoffgehalt des Einsatzgemischs unter Verwendung einer Regelung vorgegeben wird, wobei der Sauerstoffgehalt des Einsatzgemischs unter Verwendung zumindest einer dritten Messeinrichtung erfasst und/oder aus Prozessparametern abgeleitet wird.

7. Verfahren nach Anspruch 1, bei dem die Folgedaten einen über den Erfassungszeitraum ermittelten Wert des ersten und/oder des zweiten Restsauerstoffgehalts und/oder eine Schwankungsbreite hiervon und/oder einen über den Erfassungszeitraum ermittelten Wert der Betriebsstabilität kennzeichnenden Größe und/oder eine Schwankungsbreite hiervon umfassen.

8. Verfahren nach einem der vorstehenden Ansprüche, bei dem die Verfahrenssteuerung auf Grundlage der Folgedaten eine Anpassung wenigstens eines Betriebsparameters umfasst.

9. Verfahren nach Anspruch 8, bei dem der für die Selektivoxidation verwendete Reaktor unter Verwendung wenigstens eines Kühlmittels gekühlt wird, und bei dem der wenigstens eine Betriebsparameter eine Kühlmitteltemperatur des wenigstens einen Kühlmittels darstellt oder umfasst.

10. Verfahren nach Anspruch 9, bei dem das Kühlmittel im Gleich- oder Gegenstrom zu dem Einsatzgemisch durch den Reaktor geführt wird.

11. Verfahren nach Anspruch 10, bei dem der wenigstens eine Betriebsparameter einen Sauerstoffgehalt des Einsatzgemischs darstellt oder umfasst.

12. Verfahren nach Anspruch 11, bei dem der für die Selektivoxidation verwendete Reaktor eine Vielzahl von Reaktionsrohren aufweist, wobei die wenigstens eine erste Messeinrichtung und/oder die wenigstens eine zweite Messeinrichtung einem oder einem Teil der Reaktionsrohre zugeordnet ist.

13. Verfahren nach einem der vorstehenden Ansprüche, bei dem der Produktkohlenwasserstoff Ethylen und der Einsatzkohlenwasserstoff Ethan ist, und bei dem die Selektivoxidation als oxidative Dehyrierung unter Verwendung eines zumindest die Elemente Molybdän, Vanadium, Niob und optional Tellur enthaltenden Katalysators durchgeführt wird.

14. Anlage zur Erzeugung eines Produktkohlenwasserstoffs, die dafür eingerichtet ist, einen Einsatzkohlenwasserstoff in einem den Einsatzkohlenwasserstoff und Sauerstoff enthaltenden Einsatzgemisch unter Erhalt eines den Produktkohlenwasserstoff und Wasser enthaltenden Produktgemischs einer Selektivoxidation zu unterwerfen, und die dafür eingerichtet ist, unter Abtrennung zumindest eines Teils des Wassers aus zumindest einem Teil des Produktgemischs ein Folgegemisch zu bilden, wobei die Anlage dafür eingterichtet ist, das Verfahren derart durchzuführen, dass der in dem Einsatzgemisch enthaltene Sauerstoff bei der Selektivoxidation teilweise umgesetzt wird, so dass das Produktgemisch einen ersten Restsauerstoffgehalt und das Folgegemisch einen zweiten Restsauerstoffgehalt aufweist, **dadurch gekennzeichnet, dass** zumindest eine erste Messeinrichtung bereitgestellt ist, die für eine Erfassung des ersten und/oder des zweiten Restsauerstoffgehalts eingerichtet ist, dass am Ende des Katalysatorbetts zumindest eine zweite Messeinrichtung bereitgestellt ist, die zur Erfassung einer eine Betriebsstabilität kennzeichnenden Größe eingerichtet ist, dass eine Prozesssteuerungs- und/oder Auswerteeinheit bereitgestellt ist, die dazu eingerichtet ist, über einen Erfassungszeitraum Messdaten der wenigstens einen ersten Messeinrichtung und/oder der wenigstens einen zweiten Messeinrichtung zu erfassen und unter Erhalt von Folgedaten auszuwerten und/oder zu verarbeiten, und die Anlage dafür eingerichtet ist, auf Grundlage der Folgedaten eine Verfahrenssteuerung durchzuführen.
